Europäisches Patentamt

European Patent Office

Office européen des brevets

(19)

(11) Veröffentlichungsnummer: **0 193 882**
**B1**

(12) # EUROPÄISCHE PATENTSCHRIFT

(45) Veröffentlichungstag der Patentschrift:
16.05.90

(51) Int. Cl.⁵: **C07K 7/06, C07K 1/14,**
**A61K 37/02, C02F 1/62**

(21) Anmeldenummer: 86102592.2

(22) Anmeldetag: 28.02.86

(54) **Cysteinreiche, gamma-Glutaminsäure-Einheiten aufweisende Peptide, Verfahren zu deren Herstellung und ihre Verwendung.**

(30) Priorität: 01.03.85 DE 3507315
19.08.85 DE 3529625

(43) Veröffentlichungstag der Anmeldung:
10.09.86 Patentblatt 86/37

(45) Bekanntmachung des Hinweises auf die Patenterteilung:
16.05.90 Patentblatt 90/20

(84) Benannte Vertragsstaaten:
AT BE CH DE FR GB IT LI NL

(56) Entgegenhaltungen:
SCIENCE, Band 230, Nr. 4726, 8. November 1985,
Seiten 674-676; E. GRILL et al.: "Phytochelatins: The
prinicpal heavy-metal complexing peptides of higher
plants"
AGRIC. BIOL. CHEM., Band 49, Nr. 1, 1985, Seiten 71-83;
N. KONDO et al.: "Synthesis of metallothionein-like
peptides cadystin A and B occurring in a fission yeast,
and their isomers"

(73) Patentinhaber: **Consortium für elektrochemische**
**Industrie GmbH, Zielstattstrasse 20,**
**D-8000 München 70(DE)**

(72) Erfinder: **Grill, Erwin, Dipl.-Biologe,**
**Saumweberstrasse 14, D-8000 München 60(DE)**
Erfinder: **Winnacker, Ernst-Ludwig, Prof. Dr. Dipl.-Chem.,**
**Dall'Armistrasse 41a, D-8000 München 19(DE)**
Erfinder: **Zenk, Meinhart, Prof. Dr. Dipl.-Biologe,**
**Pfeivestlstrasse 17, D-8000 München 60(DE)**

**Beschreibung**

Die Erfindung betrifft cysteinreiche, γ-Glutamin-Säure-Einheiten aufweisende Peptide.

Cysteinreiche Proteine tierischen Ursprungs (Metallothioneine) sind bereits bekannt geworden. Die Aminosäuren dieser Proteine sind durch α-Peptidbindung miteinander verknüpft. Diese Metallothioneine können erhebliche Mengen an Schwermetall binden und dienen so vermutlich im entsprechend belasteten Organismus zur Entgiftung.

(Hierzu Kägi J. Biochem. 89, 1839, 1981).

Weiterhin sind auch bereits gemäß Agric. Biol. Chem. 49, 71, 1985, aus Spalthefe gewonnene Peptide der Formel H-γ-Glu-Cys-γ-Glu-Cys-Gly-OH
H-γ-Glu-Cys-γ-Glu-Cys-γ-Glu-Cys-Gly-OH
bekannt geworden.

Gegenstand der Erfindung sind Peptide, im folgenden auch als Phytochelatine bezeichnet, die durch die folgende Aminosäuresequenz charakterisiert sind:

$NH_3{}^{2+}$ - $(\gamma\text{-GluCys})_n$ Gly - COO -,
wobei n eine ganze Zahl von 4-7 ist.

γ-Glu steht für γ-Glutaminsäure, Cys steht für Cystein, Gly steht für Glycin.

Die erfindungsgemäßen Peptide können durch die folgenden Strukturformeln dargestellt werden:

n = 4γGlu-Cys-γ-Glu-Cys-γ-Glu-Cys-γ-Glu-Cys-Gly

n = 5γGlu-Cys-γ-Glu-Cys-γ-Glu-Cys-γ-Glu-Cys-γ-Glu-Cys-Gly

n = 6γGlu-Cys-γ-Glu-Cys-γ-Glu-Cys-γ-Glu-Cys-γ-Glu Cys-γ-Glu-Cys-Gly

n = 7γGlu-Cys-γ-Glu-Cys-γ-Glu-Cys-γ-Glu-Cys-γ-Glu Cys-γ-Glu-Cys-γ-Glu-Cys-Gly

Gegenstand der Erfindung sind ferner Phytochelatine mit einem Gehalt an Metallkationen. Grundsätzlich kommen alle Metallkationen in Betracht, die mit den Thiolatgruppen des entsprechenden Phytochelatins eine koordinative Bindung eingehen können. Beispiele hierfür sind insbesondere die Kationen der Metalle Blei, Zinn, Wismut, Titan, Vanadin, Molybdän, Mangan, Kobalt, Nickel, Eisen, Kupfer, Silber, Gold, Platin, Palladium, Zink, Cadmium, Quecksilber, Uran, Arsen, Selen.

Der Metallgehalt ist naturgemäß abhängig von der Wertigkeit des koordinativ an die Thiolatgruppen gebundenen Kations. Er beträgt 1 bis 4 Mol, bei zweiwertigen Kationen normalerweise 2 Mol, pro Mol Phytochelatin.

Die erfindungsgemäßen Phytochelatine sind erhältlich durch Extraktion pflanzlichen Materials. Grundsätzlich kommen als pflanzliches Material sowohl die niederen als auch die höheren Pflanzen in Betracht. Vorzugsweise werden jedoch die Angiospermen und insbesondere, wegen der leichten Zugänglichkeit von Zellkulturen, die dikotylen Angiospermen bevorzugt.

Beispielhaft genannt sei: Pflanzenmaterial aus der Familie der Apocynaceae, insbesondere Catharantus roseus, Rauwolfia serpentina, Rhazya stricta;

aus der Familie der Berberidaceae, insbesondere Berberis stolonifera;

aus der Familie der Brassicaceae, insbesondere Thlaspi alpestre;

aus der Familie der Caryophyllaceae, insbesondere Silene cucubalus;

aus der Familie der Papaveraceae, insbesondere Fumaria parviflora;

aus der Familie der Ranunculaceae, insbesondere Thalictrum dipterocarpum;

aus der Familie der Rubiaceae, insbesondere Galium mollugo;

aus der Familie der Rosaceae, insbesondere Rosa canina, Malus silvestris;

aus der Familie der Solanaceae, insbesondere Solanum marginatum, Nicotiana rustica;

aus der Familie der Asteraceae, insbesondere Echinacea purpurea;

aus der Familie der Apiaceae, insbesondere Anethium graveoleus.

Als pflanzliches Material werden vorzugsweise Zellkulturen der genannten Pflanzen eingesetzt. Es können jedoch auch die differenzierten Pflanzen oder Pflanzenteile zur Gewinnung der erfindungsgemäßen Peptide herangezogen werden.

Die Bildung der Phytochelatine in der Pflanze wird durch Behandeln der Pflanzen mit Metall bzw. mit Metallsalzen induziert. Diese Behandlung kann in jeder geeigneten Weise erfolgen, in der die Pflanzen die Metalle bzw. die Metallkationen aufnehmen können. Vorzugsweise erfolgt die Induzierung der Phytochelatine durch Behandeln von Zellkulturen mit Metallsalzlösungen in wäßrigen System. Üblicherweise wird dabei so vorgegangen, daß das Zellmaterial in der Metallsalzlösung kultiviert wird, wobei Metallkonzentrationen eingehalten werden, die unterhalb der akuten Phytotoxizität liegen. Die Untergrenze der Metallkonzentrationen liegt bei 1 μmol pro Liter. Die Obergrenze hängt, wie bereits erwähnt, stark von der Phytotoxizität des entsprechenden Metallkations ab. Sie kann bis zu 100 mmol pro Liter betragen. Bevorzugt ist der Bereich von 10 μmol pro Liter - 1 mmol pro Liter.

Vorzugsweise wird deshalb solches pflanzliches Material zur Gewinnung der Phytochelatine eingesetzt, das, wie vorstehend beschrieben, in Gegenwart von Metallen bzw. Metallkationen kultiviert wurde.

Die Extraktion des pflanzlichen Materials erfolgt nach Methoden, nach denen auch bereits bisher Peptide aus Pflanzen extrahiert werden konnten. Üblicherweise wird so vorgegangen, daß die Zellstruktur der Pflanzen oder der Zellkulturen zerstört wird. Hierzu wird das pflanzliche Material zweckmäßigerweise eingefroren, z.B. mit flüssigem Stickstoff ggf. zerkleinert und schließlich in einer wäßrigen, leicht alkalischen Zubereitung aufgenommen. Zweckmäßigerweise beträgt der pH-Wert der wäßrigen Zubereitung 7,5 - 9,5. Es können jedoch auch saure Zubereitungen verwendet werden von pH 1 - 3. Oftmals wer den als wäßrige Zubereitungen für die genannten pH-Bereiche geeignete Pufferlösungen verwendet.

Ggf. nach Abtrennen nicht aufgeschlossenen Pflanzenmaterials erfolgt die Aufarbeitung des Extrakts. Zweckmäßigerweise wird der Extrakt zunächst eingeengt. Dies kann beispielsweise durch Gefriertrocknen oder durch Absorption der Peptide an einem Ionenaustauscher und anschließendes Eluieren bewerkstelligt werden.

Danach werden die mitextrahierten Proteine durch Zusatz von Elektrolyt (zum Beispiel Ammoniumsulfat-Lösung) gefällt. Die erfindungsgemäßen Peptide verbleiben dabei in Lösung. Schließlich können weitere Reinigungsschritte erfolgen, wie beispielsweise Ultrafiltration, Gelfiltration oder Fällung der Phytochelatine mit Kupfersulfat-Lösung. Die Auswahl der Eluate kann jeweils anhand von UV-Messungen nach Maßgabe der typischen Absorptionsbande für die Metall-Thiolatbindung sowie ggf. durch qualitativen Nachweis der SH-Funktionen (beispielsweise mit Ellman's Reagenz), erfolgen.

Sofern das pflanzliche Material im basischen Medium aufgenommen wurde, fallen die Phytochelatine mit einem entsprechenden Gehalt an Metallionen an. Die freien Peptide können beispielsweise durch Ansäuern und Fällung (z.B. $H_2S$-Fällung) der Metallkationen erhalten werden. Alternativ können die mit Metallkationen beladenen Peptide beispielsweise auch mit Hilfe von Komplexbildnern (zum Beispiel Ethylendiamintetraacetat) vom Metallgehalt befreit werden.

Wird das Pflanzenmaterial in sauren Zubereitungen aufgenommen, fallen die Peptide ohne Metallkationen-Gehalt an.

Die erfindungsgemäßen Peptide der Formel
$NH_3^+$ -($\gamma$-Glu Cys)$_n$ Gly-COO-
fallen in der Regel als Gemische an, wobei das Peptid $NH_3^+$ - ($\gamma$-Glu Cys)$_4$ Gly-COO-
unter den erfindungsgemäßen Peptiden die Hauptkomponente ist. Ihr Anteil beträgt zumeist das 3 - 8-fache der höherkettigen Peptide.

Die typische Häufigkeitsverteilung der erfindungsgemäßen Peptide im Peptidgemisch, bezogen auf die Gesamtmenge erfindungsgemäßer Peptide, ergibt sich aus den folgenden Angaben:
70 - 90 mol% ($\gamma$-Glu Cys)$_4$ Gly
5 - 15 mol% ($\gamma$-Glu Cys)$_5$ Gly
2 - 10 mol% ($\gamma$-Glu Cys)$_6$ Gly
0.1 - 5 mol% ($\gamma$-Glu Cys)$_7$ Gly
Weiterhin können die Gemische, was bei den vorstehend aufgeführten mol%-Angaben unberücksichtigt blieb, analoge kurzkettige Peptide enthalten, insbesondere ($\gamma$-Glu-Cys)$_3$ Gly und ($\gamma$-Glu Cys)$_2$ Gly.

Falls erwünscht, können die Peptide nach an sich bekannten Methoden der Peptid-Auftrennung getrennt werden. Beispiele hierfür sind die HPL-Chromatograhie, die Gelfiltration, Chromatographie an Ionenaustauschern u.a.

Die erfindungsgemäßen Peptide können einzeln oder im Gemisch in Substanz oder in Form von Zubereitungen zur Anwendung kommen, die auch bereits bisher für die Formulierung von Peptid-Zusammensetzungen verwendet werden konnten. Beispielsweise können die erfindungsgemäßen Peptide in an sich bekannter Weise an Trägermaterialien physisorbiert oder chemisch adsorbiert werden.

Beispiele für Trägermaterialien sind Alginate, funktionelle Gruppen (wie zum Beispiel Oxirangruppe) aufweisende Agarosen, Zellulose, Polyacrylharze, Gläser, Silikate u.a.

Die erfindungsgemäßen Peptide finden Verwendung in Form von pharmazeutischen Zubereitungen zur Behandlung akuter und chronischer Schwermetallvergiftungen. Andererseits können auch mit Metallkationen beladene erfindungsgemäße Peptide zur Behandlung von Metallmangelerscheinungen bzw. zur Aufrechterhaltung der Homeostasis der entsprechenden Metalle (zum Beispiel Eisen, Zink) verwendet werden.

Weitere Anwendungen ergeben sich auf dem Sektor des Umweltschutzes, beispielsweise zur Klärung von schwermetall-belasteten Abwässern.

Ferner können die erfindungsgemäßen Peptide zur Anreicherung wertvoller Metalle aus wäßrigen Lösungen eingesetzt werden.

Weiterhin können schwermetall-beladene, erfindungsgemäße Peptide zur Gewinnung von entsprechenden Antikörpern eingesetzt werden, die ihrerseits wiederum als Diagnostikum für den Belastungsgrad an Schwermetallen bei Pflanzen dienen. Die Erfindung wird nun anhand von Beispielen näher erläutert:

Beispiel 1:

1.000 g (entsprechend 33 g Trockengewicht) einer Zellkultur von Rauwolfia serpentina wurden in 3 l einer 300 µmolaren wäßrigen Cadmiumsulfat-Lösung 5 Tage unter ständigem Schütteln kultiviert. Danach wurde die Zellkultur geerntet, mit destilliertem Wasser gewaschen und in flüssigem Stickstoff eingefroren. Die derart aufgeschlossene Zellkultur wurde anschließend in 500 ml Pufferlösung (10 mmolare wäßrige Lösung von Trihydroxymethylaminomethan/HCl/2-Mercaptoethanol) vom pH = 8,6 suspendiert.

Die Suspension wurde zentrifugiert. Der Überstand wurde erneut auf den pH von 8,6 gebracht und soweit verdünnt, daß die Leitfähigkeit < 1 kS betrug. Danach wurde der Überstand über eine Anionenaustauschersäule (Diethylaminoethyl-Agarose-Säule) geschickt.

Anschließend wurde mit einer wäßrigen Lösung (0,5 mol/l NaCl, 10 mmol/l Trihydroxymethylaminomethan/HCl, pH = 8,6) eluiert. Das Eluat wurde zur Proteinfällung bis zur 85%igen Sättigung mit festem Ammoniumsulfat versetzt. Danach wurde zentrifugiert und der Überstand durch Ultrafiltration (Membran YM-2 der Firma Amicon) von Ammoniumsulfat befreit und auf 15 ml eingeengt.

Schließlich wurde die eingeengte Lösung einer Gelfiltration (Sephadex® G-50, Pharmacia Uppsala, Schweden) bei pH = 7 unterworfen. Der pH-Wert wurde mit Ammoniumacetat entsprechend eingestellt. Durch Eluieren mit 5 mmolarer Ammoniumacetat-Lösung wurden zunächst ein kleiner Anteil hochmolekularen Proteins und anschließend die gewünschten Peptide erhalten.

Nach Lyophilisieren des Eluats wurden 200 mg an Peptidgemisch erhalten. Der Cadmiumgehalt betrug 15,7 Gew.-%.

**Beispiel 2:**

100 mg des gemäß Beispiel 1 erhaltenen Peptidgemisches wurden in 2 ml 0,01 normaler Salzsäure gelöst. In diese Lösung wurde gasförmiger Schwefelwasserstoff unter Fällung von Cadmiumsulfid eingeleitet. Danach wurde abzentrifugiert und der Überstand lyophilisiert. Es fielen 84 mg an metallfreiem Peptidgemisch an.

Durch HPL-Chromatographie in einer "reversed phase-Säule" (Nucleosil® C-18, 10 μm, Macherey & Nagel, Düren, DE) wurde das Peptidgemisch aufgetrennt. Es hatte folgende Zusammensetzung:
44,9 mol% $NH_3^+$ - ($\gamma$-Glu Cys)$_3$ Gly-COO-
47,6 mol% $NH_3^+$ - ($\gamma$-Glu Cys)$_4$ Gly-COO-
5,6 mol% $NH_3^+$ - ($\gamma$-Glu Cys)$_5$ Gly-COO-
1,7 mol% $NH_3^+$ - ($\gamma$-Glu Cys)$_6$ Gly-COO-
0,1 mol% $NH_3^+$ - ($\gamma$-Glu Cys)$_7$ Gly-COO-
0,1 mol% Rundungsfehler

**Beispiel 3:**

50 mg des metallfreien Peptidgemisches (gemäß Beispiel 2) wurden in 2 ml 0,01 normaler Salzsäure gelöst und mit 250 μmol Eisensulfat in Form einer 1 molaren Lösung versetzt. Es wurde unter Stickstoff-Atmospäre gearbeitet. Danach wurde mit 0,01 normaler Natronlauge neutralisiert (pH = 7,5). Die so erhaltene Lösung wurde schließlich einer Agarose-Säule (Sephadex G-25, Firma Pharmacia, Uppsala, Schweden) aufgegeben. Nach Eluieren mit mit Wasser und Lyophilisieren des Eluats wurde mit Eisen beladenes Peptidgemisch in einer Ausbeute von 50 mg erhalten.

**Patentansprüche für die Vertragsstaaten: BE, CH, DE, FR, GB, IT, LI, NL**

1. Peptide, **gekennzeichnet** durch die folgende Aminosäuresequenz:
$NH_3^+$ - ($\gamma$-Glu Cys)$_n$ Gly-COO-
wobei n eine ganze Zahl von 4 - 7 ist.

2. Peptid nach Anspruch 1, **gekennzeichnet** durch einen Gehalt an Metallkationen von 1 bis 4 mol, pro mol Peptid.

3. Verfahren zur Herstellung einer Zusammensetzung aus Peptid(en) der Aminosäuresequenz
$NH_3^+$-($\gamma$-Glu Cys)$_n$ Gly-COO-
wobei n eine ganze Zahl von 4 bis 7 ist, mit einem Gehalt an Metallkationen von 1 bis 4 Mol pro Mol Peptid, dadurch gekennzeichnet, daß
a) Pflanzenmaterial unter Einwirkung von Metallsalzen kultiviert wird und
b) das gemäß a) gewonnene Pflanzenmaterial extrahiert wird.

4. Verfahren zur Herstellung von Peptid(en) der Aminosäuresequenz
$NH_3^+$-($\gamma$-Glu Cys)$_n$Gly-COO-,
wobei n eine ganze Zahl von 4 bis 7 ist, dadurch gekennzeichnet, daß die durch das Verfahren nach Anspruch 3 erhältliche Zusammensetzung durch Fällungs- und/oder Komplexbildungsreaktion von ihrem Metallgehalt befreit wird.

5. Verwendung des Peptids nach Anspruch 1 zur Entfernung von Schwermetallen aus schwermetallbelasteten chemischen oder physiologischen Systemen.

6. Verwendung des Peptids gemäß Anspruch 2 zur Aufrechterhaltung der Homeostasis in physiologischen Systemen.

**Patentansprüche für den Vertragsstaat: AT**

1. Verfahren zur Herstellung einer Zusammensetzung aus Peptid(en) der Aminosäuresequenz
$NH_3^+$ - ($\gamma$-Glu Cys)$_n$ Gly-COO-
wobei n eine ganze Zahl von 4 bis 7 ist, mit einem Gehalt an Metallkationen von 1 bis 4 Mol pro Mol Peptid, **dadurch gekennzeichnet,** daß
a) Pflanzenmaterial unter Einwirkung von Metallsalzen kultiviert wird und
b) das gemäß a) gewonnene Pflanzenmaterial extrahiert wird.

2. Verfahren zur Herstellung von Peptid(en) der Aminosäuresequenz
$NH_3^+$ - ($\gamma$-Glu Cys)$_n$ Gly-COO-,
wobei n eine ganze Zahl von 4 bis 7 ist, **dadurch gekennzeichnet,** daß die durch das Verfahren nach Anspruch 1 erhältliche Zusammensetzung durch Fällungs- und/oder Komplexbildungsreaktion von ihrem Metallgehalt befreit wird.

3. Verwendung des durch das Verfahren nach Anspruch 2 erhältlichen Peptids bzw. Peptidgemisches zur Entfernung von Schwermetallen aus schwermetall-belasteten chemischen oder physiologischen Systemen.

4. Verwendung der nach dem Verfahren nach Anspruch 1 erhältlichen Zusammensetzung zur Aufrechterhaltung der Homeostasis in physiologischen Systemen.

**Claims for the contracting states: BE, CH, DE, FR, GB, IT, LI, NL**

1. Peptides characterized by the following amino acid sequence:
$NH_3^+$-($\gamma$-Glu Cys)$_n$ Gly-COO-
where n is an integer from 4-7.

2. Peptide according to Claim 1, characterized in that it contains 1 to 4 moles of metal cations per mole of peptide.

3. Process for the preparation of a composition of a peptide (peptides) of the amino acid sequence
$NH_3^+$-($\gamma$-Glu Cys)$_n$ Gly-COO-
where n is an integer from 4 to 7, containing 1 to 4 moles of metal cations per mole of peptide, characterized in that
a) plant material is cultured under the influence of metal salts and
b) the plant material obtained in a) is extracted.

4. Process for the preparation of a peptide (peptides) of the amino acid sequence
$NH_3^+$-($\gamma$-Glu Cys)$_n$ Gly-COO-,
where n is an integer from 4 to 7, characterized in that the composition which can be obtained by the process of Claim 3 is freed from its metal content by a precipitation reaction and/or chelating reaction.

5. Use of the peptide of Claim 1 for removing heavy metals from chemical or physiological systems which are contaminated with heavy metals.

6. Use of the peptide according to Claim 2 for maintaining the homeostasis in physiological systems.

**Claims for the contracting state: AT**

1. Process for the preparation of a composition of a peptide (peptides) of the amino acid sequence

$NH_3^+-(\gamma\text{-Gly Cys})_n \text{ Gly}-COO-$

where n is an integer from 4 to 7, containing 1 to 4 moles of metal cations per mole of peptide, characterized in that
a) plant material is cultured under the influence of metal salts and
b) the plant material obtained in a) is extracted.

2. Process for the preparation of a peptide (peptides) of the amino acid sequence

$NH_3^+-(\gamma\text{-Glu Cys})_n \text{ Gly}-COO-$,

where n is an integer from 4 to 7, characterized in that the composition which can be obtained by the process of Claim 1 is freed from its metal content by a precipitation reaction and/or chelating reaction.

3. Use of the peptide or peptide mixture which can be obtained by the process of Claim 2, for removing heavy metals from chemical or physiological systems contaminated with heavy metals.

4. Use of the composition which can be obtained by the process of Claim 1, for maintaining the homeostasis in physiological systems.

**Revendications pour les Etats Contractants: BE, CH, DE, FR, GB, IT, LI, NL**

1. Peptides, caractérisés par la séquence d'acides aminés suivante:

$NH_3^+-(\gamma\text{-Glu Cys})_n \text{ Gly}-COO-$

dans laquelle n est un nombre entier de 4 à 7.

2. Peptide selon la revendication 1, caractérisé par une teneur en cations métalliques de 1 à 4 moles par mole de peptide.

3. Procédé de production d'une composition de peptide(s) à séquence d'acides aminés suivante:

$NH_3^+-(\gamma\text{-Glu Cys})_n \text{ Gly}-COO-$

dans laquelle n est un nombre entier de 4 à 7, avec une teneur en cations métalliques de 1 à 4 moles par mole de peptide, caractérisé en ce que
a) on cultive une matière végétale en faisant agir des sels métalliques et,
b) on extrait la matière végétale obtenue selon a).

4. Procédé de production de peptide(s) à séquence d'acides aminés suivante:

$NH_3^+-(\gamma\text{-Glu Cys})_n \text{ Gly}-COO-$

dans laquelle n est un nombre entier de 4 à 7, caractérisé en ce qu'on élimine le métal de la composition obtenue par le procédé selon la revendication 3, par réaction de précipitation et/ou par réaction de complexage.

5. Utilisation du peptide selon la revendication 1 pour éliminer des métaux lourds de milieux chimiques ou physiologiques chargés de métaux lourds.

6. Utilisation du peptide selon la revendication 2 pour maintenir l'homéostasie dans des milieux physiologiques.

**Revendications pour l'Etat Contractant: AT**

1. Procédé de production d'une composition de peptide(s) à séquence d'acides aminés suivante:

$NH_3^+-(\gamma\text{-Glu Cys})_n \text{ Gly}-COO-$

dans laquelle n est un nombre entier de 4 à 7, avec une teneur en cations métalliques de 1 à 4 moles par mole de peptide, caractérisé en ce que
a) on cultive une matière végétale en faisant agir des sels métalliques et,
b) on extrait la matière végétale obtenue selon a).

2. Procédé de production de peptide(s) à séquence d'acides aminés suivante:

$NH_3^+-(\gamma\text{-Glu Cys})_n \text{ Gly}-COO-$

dans laquelle n est un nombre entier de 4 à 7, caractérisé en ce qu'on élimine le métal de la composition obtenue par le procédé selon la revendication 1, par réaction de précipitation et/ou par réaction de complexage.

3. Utilisation du peptide ou du mélange de peptides obtenu par le procédé selon la revendication 2 pour éliminer des métaux lourds de milieux chimiques ou physiologiques chargés de métaux lourds.

4. Utilisation de la composition obtenue par le procédé selon la revendication 1 pour maintenir l'homéostasie dans des milieux physiologiques.